# EUROPEAN PATENT SPECIFICATION

(11) **EP 4 023 263 B1**
(45) Date of publication and mention of the grant of the patent: **27.03.2024**
(21) Application number: 20397520.6
(22) Date of filing: 31.12.2020
(51) Int. Cl.: A61L 27/04, A61L 27/42, A61L 27/58, A61L 31/02, A61L 31/12, A61L 31/14, A61L 27/44, C22C 23/00, C22C 23/04, C22F 1/06

(54) **AN IMPLANT COMPRISING MAGNESIUM ALLOY AND A METHOD FOR PREPARING THEREOF**
IMPLANTAT MIT MAGNESIUMLEGIERUNG UND VERFAHREN ZUR HERSTELLUNG DAVON
IMPLANT COMPRENANT UN ALLIAGE DE MAGNÉSIUM ET SON PROCÉDÉ DE PRÉPARATION

(43) Date of publication of application: 06.07.2022
(73) Proprietor: BIORETEC OY, 33720 Tampere (FI)
(72) Inventor: Lähteenkorva, Kimmo, 33820 Tampere (FI); Lehtonen, Timo, 33720 Tampere (FI); Lyyra, Inari, 33720 Tampere (FI)
(74) Representative: Berggren Oy

(56) References cited:
- EP-A1- 3 693 481
- WO-A1-2014/159328
- CN-A- 108 950 336
- XIAOLING LIU ET AL: "Mechanical, degradation and cytocompatibility properties of magnesium coated phosphate glass fibre reinforced polycaprolactone composites", JOURNAL OF BIOMATERIALS APPLICATIONS., vol. 29, no. 5, 15 July 2014 (2014-07-15), pages 675-687, XP55666427, US ISSN: 0885-3282, DOI: 10.1177/0885328214541302

## Description

### Technical field

The present application relates to implants comprising magnesium alloys and methods for preparing thereof. More particularly the present application relates to magnesium alloys comprising calcium, zinc, iron, and zirconium.

### Background

Magnesium and alloys thereof are used in medical devices installed into the body, such as implants and the like. Such magnesium materials may contain additional compounds, such as other metals, rare earth metals and other compounds. Some of these compounds are not desired in such materials and may be considered impurities.

Most of the currently used magnesium alloys contain rare earth metals, such as yttrium (Y), gadolinium (Gd) and neodymium (Nd) and this is also the case in the implants commercially available in Europe. Rare earth metals are added to the alloy to strengthen it, make it more ductile and improve its corrosion behavior. Using rare earth metals in the orthopedic implants however is turned out to be very problematic. They do not naturally exist in the human body. The body has no proper means to get rid of them, so they tend to remain in the body for an extended period or even for an indefinite time. One example of such compound is gadolinium, which has been recently associated with disorders such as nephrogenic systemic fibrosis.

Magnesium may contain other compounds considered as impurities as well. Generally known impurity limits for medical purpose rare earth metal free magnesium alloy are: <30 ppm for Fe, <20 ppm for Cu, <5 ppm for Ni, <200 ppm for Mn, and <200 ppm for Si whereas the total amount of these impurities should be below 400 ppm. Magnesium is prone to corrosion, which is accelerated by galvanic coupling with impurities. Especially iron as an impurity causes localized corrosion of the magnesium material called pitting and has therefore not been desired, especially in medical products and materials.

Iron is usually already present in the raw material used for preparing magnesium alloys. Further, iron is released from steel tools used for processing the alloys, especially when sodium is present.

CN108950336A discloses a highly plastic degradable Mg-Zn-Zr-Ca-Fe alloy which can be applied to a biological implant material and a preparation method thereof. The Mg-Zn-Zr-Ca-Fe alloy material is composed of the following weight percentage elements: Zn 2.0-3.0%, Zr 0.5-1.0%, Ca 0.25-0.35%, Fe 0.025-0.03%, and the rest is Mg and its unavoidable impurities. In the Mg-Zn-Zr-Ca-Fe alloy and the preparation method thereof, an appropriate amount of Ca and Fe elements are added, and the grain of the material is refined by the heterogeneous nucleation of Zr and Fe and the dispersed CaMgZn phase to make the material better plastic. This applies in particular to the preparation of medical sutures and the like.

However it is challenging and laborious to prepare such materials or products containing less or none of these undesired compounds as the process requires extensive purification steps, such as by distillation, to remove the impurities, especially iron. This makes the process slow, complex and expensive. Only limited forms of products can be prepared. Therefore there is a need to simplify the production process of medical materials and products containing magnesium, and to obtain a variety of less expensive materials and products.

### Summary

The present invention is defined by the claims. Any other subject-matter falling outside the scope of the claims is provided for information purposes only. The present invention overcomes drawbacks of the prior art. It is possible to obtain rare earth metal-free magnesium alloys which exhibit high mechanical strength and low corrosion rate. The amount of certain impurities or otherwise undesired compounds can be maintained at a very low level. The presence of iron does not exhibit such harmful properties as in conventional products, but rather enhances the alloy's properties.

In the present invention it was found out that alloys of magnesium can be prepared with a simple method which does not aim to remove all the iron present in the alloy. It was surprisingly found out that when zirconium and/or zirconium compound(s)s is/are provided as processing aid during preparation of the alloy, the iron present in the alloy mixture could be stabilized in such way that corrosion of the material was prevented. There is no need to involve laborious or complex purification steps, such as distillation, to get rid of iron and to get a high purity alloy. Therefore it is possible to obtain a simple and economical preparation method of the alloy, and the price of the alloy itself can be kept at a significantly lower level. The method provides advantages over the whole production chain. The obtained stable and corrosion-free magnesium alloy can be used in medical applications, such as in implants and the like without the adverse effects due to otherwise too fast, uncontrollable and unpredictable corrosion rate and pitting, which produces cavities to the material and is problematic especially in implants having specific structures such as thin parts and wherein stability and/or controlled and predictable biodegradability is desired.

Further, it was found out that calcium, zinc and zirconium and preferably trace amounts of iron in the magnesium alloy enhanced the mechanical properties of the alloy, so there is no need to include the conventional rare earth metals in such alloys. The combination of zirconium and iron present in the alloy did not have negative impact to the mechanical and corrosion properties of the material and to implants prepared from the material.

Especially it was found that implants prepared from the magnesium alloy exhibited very low cytotoxicity and rather enhanced the viability of cells in contact with the material.

The present application provides an implant comprising biodegradable magnesium alloy consisting of magnesium and
- Ca in the range of 0.3-2 wt%,
- Zn in the range of 0.5-6 wt%,
- Fe in the range of 50-100 ppm, and
- Zr in the range of 100-900 ppm and total impurities including Fe and Zr in the range of 400-1000 ppm, optionally wherein total impurities comprise Mn, Cu, Ni, Al, Pb, Cd, and rare earth elements below 800 ppm, wherein the magnesium alloy has an average grain size of 40 µm or less.

The present application also provides a method for preparing an implant, the method comprising
- providing a biodegradable magnesium alloy object, preferably an elongated object, consisting of magnesium and
- Ca in the range of 0.3-2 wt%,
- Zn in the range of 0.5-6 wt%,
- Fe in the range of 50-100 ppm, and
- Zr in the range of 100-900 ppm and total impurities including Fe and Zr in the range of 400-1000 ppm, optionally wherein total impurities comprise Mn, Cu, Ni, Al, Pb, Cd, and rare earth elements below 800 ppm, and having an average grain size of 40 µm or less and
- forming the biodegradable magnesium alloy object into the implant.

The obtained composition can be identified by analyzing the content of iron and zirconium, and preferably the ratio thereof as well as the grain size of the magnesium alloy.

The present methods provide economical manufacturing of magnesium alloy to medical use when iron free manufacturing devices and contact surfaces are not needed. This is especially useful in manufacturing of different types of implants.

Such alloy composition of Mg-Zn-Ca has been verified to be biocompatible in preclinical and in clinical trials. All the alloy components are biocompatible and there are substantially no rare earth elements/metals. Preferably the magnesium alloy, and/or the implant, does not contain rare earth elements/metals, such as yttrium (Y), gadolinium (Gd) and/or neodymium (Nd), which may mean that the content thereof is below detection level.

The implant can be prepared with standard techniques, such as by extrusion, but also a variety of other techniques and methods can be applied. The processing of the magnesium alloy is easier with the aid of zirconium. No distillation is needed to remove impurities such as iron, nickel or copper. The magnesium alloy as a raw material enables using a variety of different preparation methods ranging from conventional mechanical processing methods of metal pieces to injection molding and additive manufacturing methods. A suitable preparation method and device can be selected according to the desired end product and end use. Therefore also a wide variety of different kinds of implants can be obtained from the starting material.

### Brief description of the figures

Figure 1 shows *in vitro* mass loss comparison (corrosion speed) of pure magnesium alloy (lower graph) and alloy comprising 69 ppm Fe and 228 ppm of Zr (upper graph) during twelve weeks
Figure 2 shows the effect of heat treatment on the mechanical properties of the MgCaZnFeZr-alloy
Figure 3 shows the effect of grain size on mechanical properties of MgCaZnFeZr-alloy (heat treated for 2 h at 160°C)
Figure 4 shows three examples (A-C) of orthopedic screws manufactured from the magnesium alloy

### Detailed description

In this specification, if any numerical ranges are provided, the ranges include also the upper and lower values. The open term "comprise" also includes a closed term "consisting of" as one option.

Unless defined otherwise, all technical and scientific terms used herein have the same meaning as commonly understood by one of ordinary skill in the art to which the invention belongs.

In this specification, except where the context requires otherwise, the words "comprise", "comprises" and "comprising" means "include", "includes" and "including", respectively. That is, when the invention is described or defined as comprising specified features, various embodiments of the same invention may also include additional features.

The phrases "percent by weight", "by weight", "% (w/w)" and "wt%" are intended to be defined as the percent by weight of the total expressed composition, unless otherwise explicitly stated. Additionally, as used herein, the terms "weight percent" and "percent by weight" may be used interchangeably and are meant to denote the weight percent (or percent by weight) based on the total expressed composition.

The term "biodegradable" refers to material which, when in contact with biological and/or natural environment, will degrade and/or decompose, either fully or partially. The degradation may be caused or accelerated by the biological and/or natural environment, and it may be controlled by selecting suitable composition and properties of the material.

The term "bioresorbable" refers to material, such as metals or alloys thereof, glass and/or ceramics, which in contact with natural environment such as soil or compost or biological tissues and/or physiological fluids will, following placement, degrade, resorb and/or absorb into the environment while maintaining its mechanical properties for a certain period of time. More particularly "bioresorbable", for example in context with metals or alloys thereof, glass and/or ceramics, may refer to such materials which degrade safely within the body.

The terms "bioresorbable", "biodegradable", "biosoluble", "bioabsorbable", biocorridible and "bioerodible", with or without prefix "bio", are often used interchangeably, and the terms may at least overlap. If applicable, the terms may be used interchangeably also herein. However the term "biodegradable" is intended to cover all the other terms as it does not specify the type of degradation or what will happen to the degradation products in the biological environment.

The terms "bioresorbable glass fiber", "bioresorbable fiber", "bioglass fiber", "controlled lifetime glass fiber", "alterable glass fiber", "glass fiber", and "fiber" may be used interchangeably herein.

The term decomposability may be defined here as (biology) to break down (organic matter) or (of organic matter) to be broken down physically and chemically by bacterial or fungal action; (chemistry) to break down or cause to break down into simpler chemical compounds or to break up or separate into constituent parts. Degradability may refer to the state or quality of being susceptible to breakdown or decomposition.

Herein and hereafter "optional" or "optionally" denotes that the subsequently described event or circumstance may but need not occur, and that the description includes instances where the event or circumstance occurs and instances in which it does not. "Comprises" or "comprising" denotes that the subsequently described set may but need not include other elements.

The present application provides a medical device, such as an implant,

comprising magnesium alloy. The magnesium alloy in the medical device may be present as such and/or it may be present as composite material together with one or more further material(s).

The implant may be any suitable medical device which is designed, arranged and/or intended to be inserted into living tissue, either fully or partially, such as into a body, for example into a bone. The implant may be used for bone-to-bone, soft tissue-to-bone, soft tissue-into-bone and/or soft tissue-to-soft tissue fixation. The implant is biocompatible with living tissue. The implant, or at least the magnesium alloy therein, is biodegradable. The implant may be or may be provided in the form of an orthopedic implant, for example a bone implant. The implant may or may not contain other parts in addition to a part comprising or consisting of the magnesium alloy disclosed herein. The optional other part(s) may or may not be biodegradable. The properties of the present magnesium alloys and object formed from the magnesium alloy are especially suitable for orthopedic implants, which benefit the enhanced mechanical and chemical properties of the material, such as bending and shear strength, elasticity, controlled and predictable biodegradability, and other properties disclosed herein.

Magnesium is considered biocompatible, biodegradable, bioresorbable and non-toxic and has been shown to increase the rate of bone formation i.e. to be osteoinductive, because magnesium is also an important ion in the formation of the biological apatites that make up the bulk of bone mineral, a key part to new bone formation. Therefore, magnesium can be classified as bioactive material. Magnesium is also known to have a positive influence on bone fragility and strength.

A problem with biocompatible medical devices and materials is the content of impurities, which often make the production of such material laborious and therefore expensive. In the case of magnesium one problem is the amount of iron in the raw material, which has an adverse effect on the corrosion of magnesium-based material, such as magnesium alloy. Such problems are especially pronounced in medical devices such as implants, which have specific structures and are intended in specific uses in demanding environment. Preparation of such devices may include specific challenges.

It was surprisingly found that when iron content is 50-100 ppm, the corrosion rate is not affected when zirconium and/or zirconium compound is/are involved in purification step in manufacturing, and then no distillation is needed to get rid of iron to get high purity alloy which is usually needed in medical use to avoid adverse events due to otherwise too fast corrosion rate. This yields economical manufacturing of magnesium alloy to medical use when iron free manufacturing devices and contact surfaces are not needed, for example in process steps involving plastic deformation and/or formation of the final product. Composition of Mg-Zn-Ca has been verified to be biocompatible in preclinical and in clinical trials.

The magnesium alloy comprises calcium, zinc, iron and zirconium compound. The magnesium alloy may comprise
- Ca in the range of 0.3-2 wt%,
- Zn in the range of 0.5-6 wt%,
- Fe in the range of 50-100 ppm, and
- Zr in the range of 100-900 ppm.

In one embodiment the magnesium alloy comprises
- Ca in the range of 0.4-0.7 wt%,
- Zn in the range of 0.5-0.7 wt%,
- Fe in the range of 50-100 ppm, and
- Zr in the range of 100-900 ppm.

In one embodiment the ratio of weight percentages Ca:(Zn-0.2) is more than 0.1.

In one embodiment the ratio of weight percentages of Fe to Zr is in the range of 1:1 to 1:20, such as 1:1 to 1:10. Zr affects two ways in the Mg-alloys. It is used as a "purification agent" to remove excess Fe in the casting process. It also remains in the alloy binding remaining iron into itself, which prevents iron-induced acceleration of the corrosion. The latter determines that there must be at least as much Zr as there is Fe. On the other hand, going above the 1:20 ratio in the Zr content does not give any benefits and the Zr starts to form harmful precipitates.

The magnesium alloy comprises total impurities in the range of 400-1000 ppm, the impurities including Fe and Zr. Other impurities include Mn, Cu, Ni, Al, Pb, Cd, and rare earth elements below 800 ppm, below 600 ppm, below 400 ppm, below 200 ppm or even below 100 ppm.

A process for preparing the implant disclosed herein is disclosed in claim 12 and may comprise
- providing magnesium alloy mixture,
- providing Zr compound,
- adding the Zr compound to the magnesium alloy mixture,
- allowing the Zr compound to react with Fe in a melt to form complex,
- allowing the complex to settle, and
- separating the settled part from the mixture. The remaining part may be formed into the implant, or into an intermediate product for preparing the implant, by using any suitable method or process.

The initially provided magnesium alloy mixture may already comprise iron and/or iron may be accumulated to the alloy in the process, for example from the equipment.

The zirconium and/or zirconium compound(s) present in the mixture precipitate(s) iron in the formed melt. The "zirconium compound", "Zr compound" and/or "Zr" as used herein may refer to zirconium or any suitable zirconium compound which can be used in the present methods and materials. The method comprises heating the mixture to a temperature high enough to obtain the melt. The temperature and the melt are maintained for a period of time required to allow the complex to form and settle (precipitate), preferably on the bottom of the reaction mixture, for example in a furnace, and preferably also to allow light impurities to rise to the surface of the mixture. The part of the mixture comprising the settled complex is removed, and preferably also the part of the mixture comprising the light impurities on the surface of the upper part of the mixture is removed. The remaining part of the mixture has a lowered amount of the impurities and it can be used as a raw material for preparing the implants or intermediate objects for preparing the implants.

The settling refers to precipitating or depositing of the complex to the bottom or lower part of the reaction mixture. The part at the bottom or lower part may be then separated by using suitable means and/or method(s). Separating may include or refer to removing the part of the mixture containing the settled part or the complex. After this part has been separated or removed, a mixture or composition remains containing substantially less iron and zirconium than originally. However, traces of both remain in the mixture or composition and can be detected thus indicating the production method. The remaining iron and zirconium are present in such amounts and in such form that they do not exhibit the conventional adverse effect causing the corrosion and they do not otherwise interfere the intended medical use or processability, storageability or other properties of the obtained magnesium alloy or products comprising thereof. The remaining part of the mixture, i.e. the processed magnesium alloy, which is mostly purified of iron but may still contain an amount of iron and zirconium. More particularly this processed magnesium alloy may comprise Fe in the range of 50-100 ppm, and Zr in the range of 100-900 ppm. The processed magnesium alloy consists of magnesium and
- Ca in the range of 0.3-2 wt%,
- Zn in the range of 0.5-6 wt%,
- Fe in the range of 50-100 ppm, and
- Zr in the range of 100-900 ppm and total impurities including Fe and Zr in the range of 400-1000 ppm, optionally wherein total impurities comprise Mn, Cu, Ni, Al, Pb, Cd, and rare earth elements below 800 ppm.

The processed magnesium alloy is formed into an object, which may be an elongated object, for example by using methods or processes disclosed herein, for example plastic deformation. The biodegradable magnesium alloy object discussed herein may refer to an object comprising or consisting of the biodegradable magnesium alloy. An implant or a blank for an implant, which is an intermediate product, may be obtained. The processed magnesium alloy may also be formed into an object having another shape, such as a plate or a sheet, or the elongated object may be further processed into a different form. The method for preparing the implant disclosed herein comprises
- providing magnesium alloy consisting of magnesium and
   - Ca in the range of 0.3-2 wt%,
   - Zn in the range of 0.5-6 wt%,
   - Fe in the range of 50-100 ppm, and
   - Zr in the range of 100-900 ppm and total impurities including Fe and

Zr in the range of 400-1000 ppm, optionally wherein total impurities comprise Mn, Cu, Ni, Al, Pb, Cd, and rare earth elements below 800 ppm, and having an average grain size of 40 µm or less, preferably as the object disclosed herein and forming the magnesium alloy into an implant.

The provider of the magnesium alloy, who may be considered as a first operator, may carry out the first part of the process, wherein the zirconium-iron complex is separated from the initial magnesium alloy, and thus obtaining processed magnesium alloy (the remaining part), wherefrom most of the zirconium-iron complexes are removed. The processed magnesium alloy is then recovered and provided to medical device manufacturing, for example as the intermediate objects, which may be the elongated objects discussed herein or other applicable objects. The processed magnesium alloy or the objects or products comprising thereof may be packed and transported to the implant manufacturer or other applicable product manufacturer who can utilize and further process the material. The processed magnesium alloy is in such a form which tolerates prolonged storing, handling and transporting of the material, so products with good quality can be prepared from the material. This manufacturer may be another (second) operator who may carry out the processing of the processed magnesium alloy and/or the objects.

However the magnesium alloy may comprise a non-uniform initial grain structure comprising large columnar grains growing in the direction of solidification. Such material may be brittle with weak grain boundaries and may contain defects such as shrinkage cavities, porosity caused by gases, and foreign material such as metallic oxides. It is possible to form or deform such material into more uniform structure exhibiting higher toughness, strength, ductility and resistance to vibration and shock. Also such deformed form exhibits better formability into implants. In such process the magnesium alloy is formed into an object having a specific grain size.

The present method may comprise heating the magnesium alloy, which may be called a first heat treatment or forming or deforming treatment. The process preferably comprises deforming the magnesium alloy. The heating is carried out at a temperature high enough to obtain a plasticly deformable form and/or grain refinement, which temperature may be least 200°C, or may be least 250°C, such as 200-400°C, or more. The magnesium alloy may be heated above the recrystallization temperature of the magnesium alloy to be able to plastically deform it in a suitable working operation. The heated magnesium alloy is then subjected to one or more working operation(s), which may be carried out with suitable method(s) and device(s), such as by extrusion, rolling, drawing and/or forging, which may be called plastic deforming operation, process or step. The process can also be characterized as a grain-refinement operation, process or step. The process may be or comprise a cold drawing or a cold deforming process. In such process a specific grain size of the material is obtained and can be controlled. This step can be carried out by a first operator, such as the manufacturer and/or the provider of the magnesium alloy, or it can be carried out by a second operator, such as the implant manufacturer. The process chain may also include more operators, which each may carry out one or more of the process steps discussed herein.

Extruded bars may have poor diameter tolerances, straightness, roundness, and surface quality. Therefore machining implants from extruded bars may be challenging, very time-consuming, and sometimes requires extra machining steps, and still, the scrap percentage remains remarkably high. Especially cannulation drilling is challenging; either the drill bit breaks or the cannulation does not stay centered. The cold drawing process can overcome these issues. In one example the Mg-alloy bar is drawn through the die, which is slightly smaller than the Mg-alloy bar. This process is repeated several times until diameter, straightness, roundness, and surface quality are on a preferable level. In each process step, the die is slightly smaller than the previous. With such method elongated objects with high quality can be prepared.

Such treatment has a remarkable impact on the mechanical properties of the obtained magnesium alloy, which is especially desired in applications requiring load-bearing properties and implants having specific structures. The heat treatment and deformation does not necessarily change the shape or the chemical composition of the alloy, at least substantially, but it rather changes the microstructure thereof, which was found especially advantageous in implant applications. This process may be a plastic deformation process. In plastic deformation processing the dislocation density of the magnesium alloy is increased, and a grain refinement effect is obtained. This results in improved strength due to resistance to dislocation movement. The properties of the material, such as grain size versus elongation, can be adjusted by selecting a suitable processing degree, such as an extrusion ratio, so that the properties of the final implant can be controlled already at this point of process. It is possible to prepare intermediate products which are designed for implant production. This process, as well as other additional process steps such as a second heat treatment step, enable controlling the grain size and other properties having an impact to the suitability of the material for implant use, such as sterility and biocompatibility. It is possible to obtain increased sterility and/or biocompatibility compared to untreated material or implants. Biocompatibility of the magnesium alloy and/or the implants, including for example cytotoxicity, can be determined by ISO10993.

In the plastic deformation process a specific grain size of the alloy is obtained, wherein the deformed magnesium alloy has an average grain size of 40 µm or less. Therefore the method may comprise (plastic) (de)forming or grain refining the heated magnesium alloy into an object, such as into an elongated object, having an average grain size of 40 µm or less. The elongated object may refer to any object which has been deformed in such way that the length of the material at least in one direction is increased. The elongated object may be a rod, a tube but also a plate, a sheet, a fiber, a filament, a wire or the like object, having a rod-like form, tube-like form, plate-like form, sheet-like form, fiber, filament or wire like form, or other similar or applicable form.

In one example the processing is carried out by using a suitable extruder so that an elongated object is formed. In one example the processing is carried out by rolling, more particularly by hot rolling.

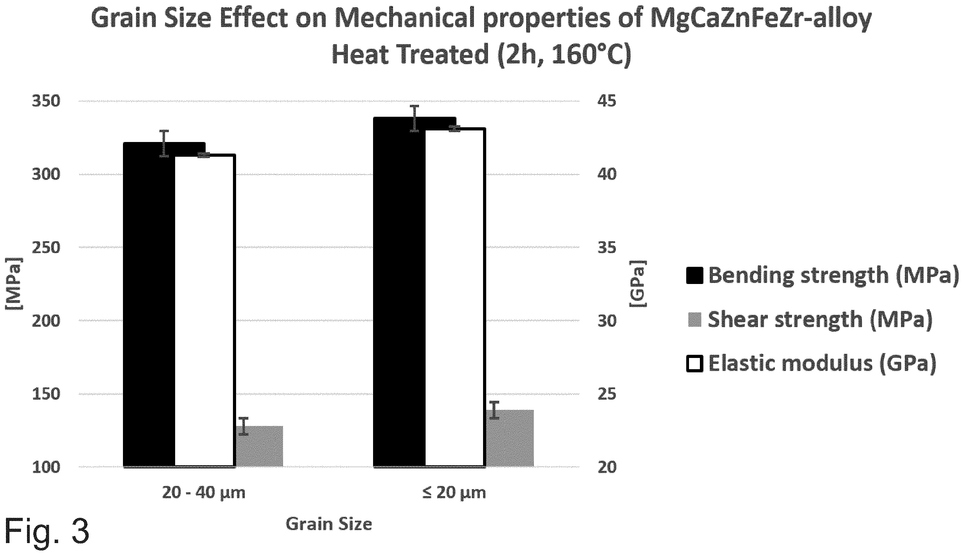

In one embodiment the magnesium alloy has an average grain size of 40 µm or less. The (plastic) deformation treatment and/or grain refinement process, and optional second heat treatment, enable obtaining the small average grain size of 40 µm or less, or 30 µm or less, or even 20 µm or less, which is considered as an indication of the high quality of the obtained magnesium alloy as well as a feature indicating the production process thereof. Such magnesium alloy having an average gran size of 40 µm or less was found to provide enhanced bending strength, shear strength and elastic modulus compared to untreated magnesium alloy, but especially an average grain size of 20 µm or less provided even better bending strength, shear strength and elastic modulus as shown in figure 3. The average grain size may be in the range of 1-40 µm, such as 5-40 µm, 10-40 µm, 1-30 µm, 5-30 µm, or 1-20 µm or 5-20 µm. The grain size refers to the smallest diameter of a grain. In the tests it was found out that ideally the grain size should be 20 µm or less, but already a magnesium alloy having a grain size of 40 µm was useful for most applications. The smaller grain size correlated with predictable corrosion rate, so especially for biodegradable implant use it is desired to obtain a grain size of 40 µm or less, as disclosed herein. The grain size may be presented as an average grain size or a number-average grain size. The grain size may be determined microscopically, for example by using optical microscopy or electron microscopy, such as scanning electron microscopy (SEM).

Optical microscopy can be carried out with any suitable method. The samples to be studied may be pretreated, such as etched, for example by using the following etching method. Two etching solutions are prepared. Etching solution 1 comprises 100 ml ethanol and 5.6 g of picric acid. Etching solution 2 comprises 5 ml of acetic acid and 5 ml deionized water The final etching solution is prepared by combining 40 ml of solution 1 and 10 ml of solution 2. Samples are polished with an oxide polishing suspension (+ammonia), cleaned and subsequently immersed in the etching solution for about 5-10 seconds. The samples are cleaned with isopropanol and dried. This etches the grain boundaries and with polarized light the grains can easily be distinguished. The average grain sizes can be determined from microscopic images.

The obtained (elongated) magnesium alloy object can be formed into a desired shape, especially into an implant or any other applicable medical device, or into a different form.

The magnesium alloy, such as an object obtained from the magnesium alloy, may be provided in any suitable form and/or processed into such form, such as solid pieces, chips, powder, granules, wire or the like, or formed into such form.

The present application provides a method for preparing an implant, the method comprising
- providing biodegradable magnesium alloy object, preferably an elongated object, consisting of magnesium and
   - Ca in the range of 0.3-2 wt%,
   - Zn in the range of 0.5-6 wt%,
   - Fe in the range of 50-100 ppm, and
   - Zr in the range of 100-900 ppm and total impurities including Fe and

Zr in the range of 400-1000 ppm, optionally wherein total impurities comprise Mn, Cu, Ni, Al, Pb, Cd, and rare earth elements below 800 ppm, and having an average grain size of 40 µm or less, and
- forming the biodegradable magnesium alloy into an implant. The biodegradable magnesium alloy, which is the processed magnesium alloy, may be obtained from the preparation method disclosed herein.

One embodiment provides providing biodegradable magnesium alloy and heating the magnesium alloy to above the recrystallization temperature of the magnesium alloy, such as to a temperature of at least 200°C, for example to a temperature in the range of 200-400°C, in the range of 200-300°C or in the range of 250-400°C, and
- forming the heated magnesium alloy into the object having the average grain size of 40 µm or less, preferably in a plastic deformation and/or grain refinement process. As discussed in previous, this process can be carried out for the initial magnesium alloy to obtain a desired grain size, plastic deformation and/or gran refinement of the magnesium alloy. This process can be carried out before packing, storing and/or transporting the material, so that objects which tolerate handling, packing, storing and/or transporting are obtained, This can be carried out for example by the magnesium alloy manufacturer or provider, which can be for example a first or second operator in the production chain. Such an operator can therefore prepare in intermediate product having good quality and for example high dimension accuracy. The intermediate product can be provided to another operator in such form that has not damaged or degraded during handling, packing, storing and/or transporting, and which can be easily further processed, for example by using any of the methods disclosed herein, and implants with good quality can be obtained. For example the intermediate product may be an elongated product, such as an rod or a tube, which has high dimensional accuracy and desired precision.

One embodiment provides a method for preparing an implant, the method comprising
- providing biodegradable magnesium alloy consisting of magnesium and
   - Ca in the range of 0.3-2 wt%,
   - Zn in the range of 0.5-6 wt%,
   - Fe in the range of 50-100 ppm, and
   - Zr in the range of 100-900 ppm and total impurities including Fe and Zr in the range of 400-1000 ppm, optionally wherein total impurities comprise Mn, Cu, Ni, Al, Pb, Cd, and rare earth elements below 800 ppm,
- heating the magnesium alloy to above the recrystallization temperature of the magnesium alloy, such as to a temperature of at least 200°C, for example to 250-400°C,
- forming the heated magnesium alloy into an object having an average grain size of 40 µm or less, and
- forming the object into the implant.

The present application discloses, but not claims, use of said magnesium alloy, or use of an object comprising said magnesium alloy, for preparing an implant, such as an implant disclosed herein with a method disclosed herein.

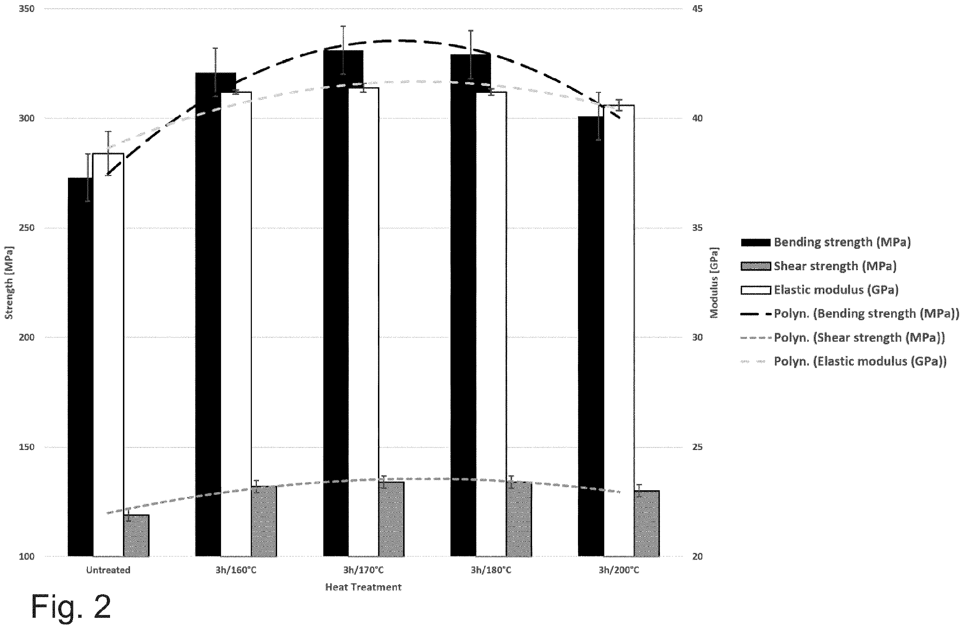

The method may comprise an additional (second) heat treatment either before or after the implant forming step by keeping the Mg-alloy at 160-200°C, preferably 160-180°C, for 30-120 minutes. This second heat treatment has an impact on the grain size of the material, and to the mechanical properties of the material and the implants obtained from the material. As shown in Figure 2, the heat treatment at the range of 160-180°C provides especially increased bending strength, shear strength and elastic modulus. This heat treatment is separate from the heat treatment carried out at the plastic deformation/grain refining step, and is carried out after the plastic deformation/grain refining. The first and/or the second heat treatment(s) may provide suitable dry heat sterilization of the magnesium alloy, which may be required for the medical uses of the implants. However the second heat treatment may be carried out at different location and/or by different operator than the first heat treatment, and it can be carried out especially to obtain the dry heat sterilization effect. The dry heat sterilization may be carried out by using any suitable heating device or means, such as a specific dry heating device. This can be carried out for example by the implant manufacturer.

In one embodiment the method comprises heating the biodegradable magnesium alloy, which may be in the form of the object, at 160-180°C for at least 30 minutes, such as 30-180 minutes, for example 30-120 minutes, before and/or after forming the biodegradable magnesium alloy into the implant. In one embodiment the method comprises heating the implant at 160-180°C for at least 30 minutes, such as 30-180 minutes, for example 30-120 minutes.

The provided magnesium alloy or the object is formed into the implant by using a suitable forming or processing method(s) and/or device(s). Different methods and devices may be applied depending on the desired end product and/or end use. Forming the object into an implant may or may not include substantial processing. In some cases the object may be already in a suitable form after previous process steps, so it may be necessary merely to clean, polish, pack and/or carry out other minor actions to obtain the final product, which is the implant, preferably ready to be used.

In one embodiment the forming the object into the implant comprises processing the object with one or more of the following:
- mechanical machining,
- laser machining,
- wroughting,
- water jet processing,
- additive manufacturing, such as comprising providing the biodegradable magnesium alloy as powder, granules or as a wire, and
- injection molding,
to form the implant.

The present disclosure provides an implant obtained with any of the methods disclosed herein.

The processing may include one or more processing steps that modify the shape of the object towards the shape of the implant, i.e. to obtain the desired shape of the final product or part thereof. Instead of implant the method may be used to produce another applicable medical device, or other device or object, or a part thereof.

Mechanical machining may comprise processing the object with one or more machining means which remove material from the object and/or change the shape of the object, for example bend the object. Machining means may comprise one or more machine tools, for example tools for forming one or more thread, groove or the like form(s) to the object, and/or other means such as pressing means.

Laser machining, such as laser beam machining, comprises processing the object with laser energy. The laser may be provided by one or more source(s) of laser, such as a gas laser, a solid state laser or an excimer.

Wroughting refers to processing the object in solid form through one or several processing steps and it may comprise different type of molding, shaping and/or manufacturing steps, such as pressure compression molding and/or profile or sheet extrusion. Wroughting may comprise heating and working with one or more tools(s) and/or machine(s). A wrought object is obtained.

Additive manufacturing, also known as 3D printing, may comprise processing the biodegradable magnesium alloy with additive manufacturing means. The biodegradable magnesium alloy may have been formed into a desired form suitable as raw material or starting material for additive manufacturing, such as powder, chip, granule or wire form. This form has the composition of the biodegradable magnesium alloy disclosed herein, but it has been processed to a form which is compatible with the additive manufacturing means. Therefore the method may comprise providing the magnesium alloy in a suitable form, such as powder, chip, granule or wire form. Such forms may be used in other manufacturing methods as well, when applicable.

Additive manufacturing means suitable for processing metal may be used. ISO/ASTM52900-15 defines seven categories of additive manufacturing processes within its meaning: binder jetting, directed energy deposition, material extrusion, material jetting, powder bed fusion, sheet lamination, and vat photopolymerization. Usually additive manufacturing methods comprise forming the object layer-by-layer, i.e. depositing the material to add a layer and repeating this.

The method may comprise providing a computer-stored 3D model of the object to be manufactured. The 3D model is then used to control the additive manufacturing means to create an object according to the 3D model.

In certain methods the material is melt or soften to produce layers. One example is Fused filament fabrication, also known as Fused deposition modeling (FDM), wherein the object is produced by extruding small beads (granules) or streams of material which harden immediately to form layers. A filament of metal wire is fed into an extrusion nozzle head (3D printer extruder), which heats the material and turns the flow on and off. FDM is somewhat restricted in the variation of shapes that may be fabricated. Another technique fuses parts of the layer and then moves upward in the working area, adding another layer of granules and repeating the process until the piece has built up. This process uses the unfused media to support overhangs and thin walls in the part being produced, which reduces the need for temporary auxiliary supports for the piece.

Examples of a suitable method for manufacturing objects from the present magnesium alloys include powder bed fusion methods, which include several processes such as DMLS, SLS, SLM, MJF and EBM. Powder Bed Fusion processes can be used with an array of materials and their flexibility allows for geometrically complex structures. The starting material may be provided as powder. One example is direct metal laser sintering (DMLS). Another example is selective laser sintering (SLS). Another example, selective laser melting (SLM) does not use sintering for the fusion of powder granules but will completely melt the powder using a high-energy laser to create fully dense materials in a layer-wise method that has mechanical properties similar to those of conventional manufactured metals. Electron beam melting (EBM) is a similar type of additive manufacturing technology. EBM manufactures parts by melting metal powder layer by layer with an electron beam in a high vacuum. With laminated object manufacturing, thin layers are cut to shape and joined together.

Another example of a suitable method is powder-fed directed-energy deposition, a high-power laser is used to melt metal powder supplied to the focus of the laser beam. The powder fed directed energy process is similar to Selective Laser Sintering, but the metal powder is applied only where the material is being added to the part at that moment.

Additive manufacturing enables preparing complex or challenging objects, which may not be possible to prepare with conventional technologies. For example the inner parts of the object may contain specific structures, which cannot be formed for example by molding. It is possible to obtain objects which are light and contain specific structures, which may be needed in specific uses, such as in specific implants. It is also possible to prepare individualized object, for example medical devices, such as implants, designed for an individual, for example according to individual properties. For example it is possible to design and prepare a bone implant to treat a specific bone fracture or other condition. This may include imaging the patient, possibly creating a 3D model of the body part in need of treatment, such as specific bone(s), creating a 3D model of a medical device compatible with the body part in need of treatment, and preparing the medical device according to the 3D model by using additive manufacturing.

Injection molding may comprise any suitable injection molding method. Especially suitable injection molding methods include thixotropic molding, liquid metal molding and metal injection molding, preferably by using a binder.

Thixotropic molding, also called Thixomat or Thixomolding according to the developing company, is a precision magnesium casting method. Thixotropic molding refers to the thixotropic nature of magnesium alloys. When heated to just the right temperature-above the melting point of some alloy components, below that of others, the alloy turns into a slurry with solid and liquid portions. The slurry's thixotropic behavior enables the metal molding process to work very much like plastic injection molding. The process may be carried out as a single step process. Room temperature magnesium alloy chips are fed into a back end of a heated barrel through a volumetric feeder. The barrel is maintained under an argon atmosphere to prevent oxidation of the magnesium chips. A screw conveyor located inside the barrel feeds the magnesium chips forward as they are heated into the semi-solid temperature range. The material may be progressively heated in the machine barrel to about 40°C below the liquidus of the alloy. The screw rotation provides the necessary shearing force to generate the globular structure needed for semi-solid casting. Once enough slurry has accumulated, the screw moves forward to inject the slurry into a steel die.

Processing temperatures up to 590°C may be used. Compared to for example conventional die-casting, thixotropic molding enables obtaining higher density and elongation, smaller minimum wall thickness and better tolerance control.

In liquid metal molding small batches of magnesium alloy are melted in a vacuum chamber to avoid contamination from oxygen. The melted alloy is poured into the shot sleeve where a plunger pushes the material into a steel mold. The liquid metal molded part comes out of the mold to net shape, great precision, and with full physical properties. Secondary processes to remove the part from the runner and overflows are usually necessary.

Metal injection molding process involves combining fine metal powders with plastic binders which allow the metal to be injected into a mold using equipment similar to standard conventional plastic injection molding machines. After the part is molded and before the binders are removed, the part is referred to as a 'green part'. The next step is to remove the binders with solvents and/or thermal processes. The resultant metal part is sintered at temperatures great enough to bind the particles but not melt the metal.

The object may be a part of the implant, so the implant may comprise the object or a further object obtained from the initially formed object. The implant may also consist of the object or an object obtained from the initially formed object. For example the object may be further processed, for example mechanically and/or chemically. In mechanical processing the object may be processed to form one or more groove(s), aperture(s), thread(s) and the like forms. In chemical processing the object may be coated with one or more coating(s), combined with one or more agent(s) or compound(s), reacted with one or more reagent(s), or combination(s) thereof. The object may be formed or included into a composite product.

The forming may comprise forming the object into an implant. The implant may be any suitable implant, such as an orthopedic implant, for example a bone implant. The orthopedic implant may be in a suitable form, and it may be formulated or processed into the desired form. The implant may be or comprise any kind of implant used for surgical musculoskeletal applications. The implant may comprise a screw, a plate, a pin, a tack or a nail, such as intramedullary nail, preferably for the fixation of bone fractures and/or osteotomies to immobilize the bone fragments for healing; a suture anchor, a tack, a bolt, a clamp, a clip, a staple, a mesh, a scaffold, a cage, a Kirschner wire, a stent or other device preferably for bone-to-bone, soft tissue-to-bone, soft tissue-into-bone and/or soft tissue-to-soft tissue fixation; such as device used for supporting tissue or bone healing or regeneration; or cervical wedge or lumbar cage or plate, or a screw preferably for vertebral fusion and other operations in spinal surgery.

In one embodiment the implant is a screw. The screw may be a trauma screw or orthopedic surgical screw. The screw may be used in fixation of broken bones or in osteotomies in small bones in extremities, in long bones like femur, tibia, fibula, humerus, radius and ulna, in cranial bones, cranio maxillofacial bones, in spine or in bones in rib cage. The screw may be fully or partially threaded. A screw may include one or more screw thread(s). Threads may have constant or variable profile i.e. profile height and other dimensions may vary. Threading may have a constant or variable pitch. The screw may be fully threaded, or it may be partially threaded (lag). The screw may have a head, or it could be headless, such as a compression screw. The screw may be cannulated or non-cannulated. The screw may be used in weight (load) bearing or non-weight bearing applications. In orthopedics, weight bearing application refers to an injured body part a person puts weight through.

In one embodiment the implant is a plate. The plate may be used in fixation of broken bones or in osteotomies in small bones in extremities, in long bones like femur, tibia, fibula, humerus, radius and ulna, in cranial bones, cranio maxillofacial bones, in spine or in bones in rib cage. The plate may be fixed into the bone with special screws, tacks or pins. Design of the plate may be for example straight or curved, L-shape, X-shape, H-shape or any other planar geometrical shape. It may be bend in certain radius beforehand or during surgery for example by surgical pliers.

In one embodiment the implant is a tack. The tack may be used in various applications, where soft tissue is fixed to the bone in joint areas like knee, shoulder, hip, ankle, wrist or in hand or foot joints (fingers and toes). Soft tissue may be for example tendon, ligament, joint capsule or cartilage tissue.

In one embodiment the implant is a pin. The pin may be used in fixation of broken bones in either weight bearing or non-weight bearing applications. The pin surface may have longitudinal or transverse grooves or ridges in order to get better fixation torsional stability, higher pullout force and better liquid flow along the implant.

In one embodiment the implant is a nail. The nail may be used in fixation of broken bones in either weight bearing or non-weight bearing applications, as well as in soft tissue to bone fixations i.e. tendon, ligament, cartilage, joint capsule. The nail surface may have longitudinal or transverse grooves or ridges in order to get better fixation torsional stability, higher pullout force and better liquid flow along the implant.

In one embodiment the implant is an intramedullary nail. Intramedullary nail, also called as intramedullary device, intramedullary rod or inter-locking nail, is a metal rod which is designed to be forced into the medullary cavity of a bone, especially to treat fractures of long bones of the body.

In one embodiment the implant is a Kirschner wire. Kirschner wire, also called as K-wire or K-pin, is a sharpened, smooth wire or pin. A Kirschner wire may be provided in different sizes and is used to hold bone fragments together (pin fixation) or to provide an anchor for skeletal traction. A Kirschner wire typically has a diameter from 0.5 to 4 mm. It may be used in fixation of broken bones in typically non-weight bearing applications or to provide an anchor for skeletal traction. It may be drilled directly through the bones in its final implantation site and it may be bend during surgery. After getting the Kirschner wire in proper depth the excess of it may be cut with surgical pliers or saw. The Kirschner wire sharp point or it may have a flute or a thread with constant or variable height, for aiding K-wire to proceed through the bone while drilling. The Kirschner wire surface may have longitudinal or transverse grooves or ridges in order to get better fixation torsional stability, higher pullout force and better liquid flow along the implant.

In one embodiment the implant is a suture anchor. The suture anchor may be used to soft tissue, like tendons and ligaments fixation to the bone by means of one to three sutures each having two limbs. Suture anchor may be partially or fully threaded, barbed or expandable a screw or a rivet type. It has an eyelet, which may be recessed or prominent, hard or suture eyelet. Anchor may have a leading edge which may be either inserted into a pre drilled hole or directly screwed into bone.

In one embodiment the implant is a interference screw. The interference screw is a direct tendon-to-bone interference fixation device. It is a compression fixation device, which relies on the screw threads to engage and compress the graft to the bone in the drill hole for fixation. Interference screw may be used in fixation of soft tissue, bone-tendon-bone or bone-tendon grafts. Graft may be auto-, allo- or xeno-graft harvested from various locations of the body. Interference screw may be self-tapping or non-self-tapping.

In one embodiment the implant is a mesh or a scaffold. The mesh or the scaffold may be or comprise foam or fibrous structure. Fibrous structure may be weaved, knitted, braided or randomly oriented fibers. It may be 3-dimensional or flat sheet type of structure. It may be used in repairing defects in bony tissue or osteochondral tissue.

In one embodiment the implant is a wedge. The wedge may be used in opening wedge osteotomy for example in proximal tibia for correcting incorrect tibial plateau angle or in foot in flat foot reconstruction surgery.

In one embodiment the implant is a spinal cage. The spinal cage may be used for serving as a space holder between affected vertebrae. It allows bone to grow through it, eventually replacing it fusing two vertebrae together. Cage may have a solid, mesh type, hollow or perforated structure.

The implant may be elongated implant having a length:diameter ratio of 2 or more, such as 4 or more, 5 or more, 8 or more, or 10 or more. The implant may be an elongated bone fixation device, i.e. an orthopedic implant. The present materials and methods suit well for preparing elongated implants, and the properties of the material are suitable for such elongated objects.

In one embodiment the implant is an orthopedic implant, which is an elongated bone fixation device having a length:diameter ratio of 2 or more and which may be selected from a screw, an intramedullary nail, a pin and a Kirschner wire, wherein the magnesium alloy has an average grain size of 40 µm or less.

The implant may consist of the magnesium alloy, especially when it is desired that the implant as a whole will degrade in the body. Alternately the implant may comprise a part obtained from the magnesium alloy, which may consist of the magnesium alloy, and further one or more other part(s), which may or may not be biodegradable.

### Composite materials

In one embodiment the implant is in a form of a composite with one or more biodegradable polymer(s), glass fiber(s), bioglass and/or ceramic material(s) such as hydroxyapatite, tricalcium phosphate, calcium carbonate, calcium sulphate, and/or is doped with silicone, magnesium etc. The implant may comprise the composite or it may consist of the composite.

The magnesium alloy is provided as a separate part having reinforcing properties, such as a rod, a plate, a core, a tube or fibers or other reinforcing shape, such as any of the shapes or forms disclosed herein. Preferably the magnesium alloy is provided as a self-standing or self-supporting form or shape. The magnesium alloy may be formed first and then combined with the other materials. Magnesium alloy as a coating is excluded.

The implant may comprise or be based on composite material comprising the magnesium alloy, which composite material may be in a form of a composite product. The composite material may be any type of applicable composite material. The process for manufacturing the composite material may be a continuous process or a batch process. The composite material may be a hybrid composite material comprising more than two types of materials.

In composite materials the main input from magnesium alloys comes from stiffness and flexural modulus. Other components of the composite may include for example glass, such as bioresorbable glass, which may be included to regulate the strength, corrosion properties and hydrogel gas evolution, and/or polymer, such as one or more type of polymer, such as plastic polymer.

In order to modify the degradation of the final implants, to enhance their surface properties, or to add biologically active compounds therein, they can be further modified by an additional resorbable polymer coating layer with a process that may include co-extrusion, dip coating, electro spraying, injection molding, solution impregnation or any other known technique used in polymer, pharmaceutical, device or textile industry. The polymers may be one or more of those mentioned herein.

The object, the composite product, or the implant, may comprise a coating and/or a surface modification. Coatings for biomaterials, especially biodegradable magnesium, have the same requirements as the base materials themselves of being biocompatible and fully degradable. The latter point is particularly salient for understanding what occurs over the medical device life cycle. In the case of magnesium, coatings themselves cannot be perfect barriers to corrosion (which would be the goal of a coating system on a structural material). To allow an magnesium-based implant to biodegrade, the coatings must, at some stage, cease to be a corrosion barrier, although they may be required to provide an effective method to reduce the initial corrosion rate of the bare metal so the surrounding bone tissue (in the case of orthopedics) may start to form. Ideally, the coating would itself degrade gradually, helping to control the overall corrosion process while leaving no harmful traces. There are a large number of possible coating technologies for magnesium biomaterials, including anodization, metal-metal coatings, plasma spray, chemical vapor deposition, atomic layer deposition, pulsed laser deposition, ion beam assisted deposition, solution, emulsion and suspension coatings, calcium phosphate (CaP) deposition achieved by various means and the well-known methods of electrodeposition and conversion coating. Moreover, the galvanic corrosion problem can only be solved by proper coating systems, but chemical conversion coatings are just a few micrometers thick and thus they are only offering a limited protection.

The object, the composite product, or the implant, may comprise bioresorbable and/or bioactive glass. Bioresorbable and bioactive glasses have a capability of reacting with physiological fluids forming tenacious bonds to bone through the formation of bone-like hydroxyapatite layers leading to effective biological interaction and fixation of bone tissue with the material surface. Moreover, in the case of silicate and phosphate bioactive glasses reactions on the material surface induce the release and exchange of critical concentrations of soluble Ca, P and Na ions, which can lead to favorable intracellular and extracellular responses promoting rapid bone formation. Bioresorbable glass compositions may be provided containing no sodium or additional elements incorporated in the silicate or phosphate network such as fluorine, magnesium, strontium, iron, silver, boron, potassium, zinc, copper, barium, lithium or combinations thereof. Fabrication techniques for bioactive glasses or glass fibers include both traditional melting methods and sol-gel techniques. The typical feature common to all bioactive glasses, being melt or sol-gel derived, is the ability to interact with living tissue, in particular forming strong bonds to bone (and in some cases soft tissue, a property commonly termed bioreactivity or bioactivity, as mentioned above. For establishing bond with bone, such a biologically active apatite, a surface layer must form at the material/bone interface. Thus, one basis of the bone bonding property of bioactive glasses is the chemical reactivity in physiological body fluids (in vitro and in vivo), which may result in the formation of a hydroxycarbonate apatite (HCA) layer to which bone can bond. Briefly, the processes on the glass surface are characterized by ion leaching/exchange, dissolution of the glass network and precipitation and growth of a calcium-deficient carbonated apatite (HCA) surface layer, whereas cellular reactions include colonization, proliferation and differentiation of relevant (bone) cells.

The bioactive glasses presented in previous exhibit several advantages in comparison to other bioactive ceramics, e.g. sintered hydroxyapatite, in tissue engineering applications. Polymer/bioceramic composite materials represent a convenient alternative due to the possibility to tailor their various properties (e.g., mechanical and structural behavior, degradation kinetics and degree of bioactivity). Composites made of polymers and bioceramics may combine the advantages of their singular components. Polymers exhibit generally high ductility, toughness, favorable formability as well as processability and plasticity. The glass or glass-ceramic phase adds stiffness and mechanical strength to the composite. In particular, composites based on biodegradable polymers may be useful as bone tissue engineering materials because this particular combination does not require a revision surgery for their removal as newly formed bone gradually substitutes the implanted material during degradation.

### Hybrid composite material

The composite material may be hybrid composite material, which may comprise three or more materials. The (hybrid) composite material may comprise (magnesium alloys combined or embedded with bioresorbable glass fiber reinforced polymer matrix. It may further comprise a coating on the surface of the magnesium or magnesium alloy, preferably to act as an adhesion layer to bioresorbable glass fiber reinforced polymer matrix and/or to act as further layer to slow down hydrogen gas evolution. It may also act as a hydrogen trap, which prevents hydrogen release from the structure, and/or it may keep corrosion products on the magnesium alloy's surface forming corrosion inhibiting layer.

The composite materials are useful as structural fixation for load-bearing purposes, exhibiting improved mechanical properties as a result of hybrid composite structure.

The hybrid composite material can minimize or even eliminate drawbacks of the prior art materials , i.e. the composite retains its strength and modulus *in vivo* for example for a time period sufficient for bone fracture healing. Indeed, high strength and flexural modulus matched with cortical bone with good strength retention *in vivo* conditions can be achieved through combining two bioresorbable reinforcement, magnesium/magnesium alloy and glass fiber into bioresorbable polymer matrix. Mechanical strength as used here includes bending strength, torsion strength, impact strength, compressive strength and tensile strength.

The hybrid composite material is malleable i.e. surgeon can shape the implant by bending it according to the bone's anatomy and shape before inserting it. Due to the hybrid composite materials' metallic, plastic nature the new shape will stay unchanged during the healing period.

The magnesium alloy is a reinforcement, and may be in a form of a rod, a plate, a core, a tube or fibers or other reinforcing shape, in general a self-supporting shape, which may be a physical form which brings a reinforcing effect to the hybrid composite and may be embedded by bioresorbable fiber reinforced polymer matrix.

According to examples, the amount of the magnesium or the magnesium alloy in the hybrid composite may be 1-99 weight-%, preferably 10-90 weight-%, more preferably 20-80 weight-%, and most preferably 30-70 weight-% of the total weight of the hybrid composite material.

Although the lower strength of Mg compared to Ti may be beneficial with respect to stress shielding, it also means that there may be a greater chance of failure in high load applications, such as the spine where compressive loads during certain activities may exceed 3500 N. It is vital to ensure that any implant is designed to sustain its load without deformation. However, this aspect is even more crucial when considering degradable materials, as an appropriate mechanical support is required throughout the entire bioresorption and bone remodeling process.

### Bioresorbable glass fiber reinforced polymer matrix

The present application provides a hybrid composite material comprising magnesium or magnesium alloy included, such as embedded, in a discontinuous or continuous bioresorbable glass fiber reinforced polymer matrix. The composite material disclosed herein preferably comprises free fiber orientation in a one or more successive layers, preferably at least glass fibers, wherein the layer comprises a bioresorbable polymer matrix and a bioresorbable reinforcing fiber or fiber bundle.

The term "free fiber orientation" refers to unrestricted choice of fiber orientation of the bioresorbable reinforcing fiber or fiber bundle of the bioresorbable glass fiber reinforced polymer matrix when designing the desired fiber orientation of the orthopedic implant. The desired fiber orientation, however, may be dependent of the requirements of the application.

The bioresorbable glass fiber may be used as continuous form as strands, roving's, yarns, tapes, textiles or chopped to form chopped strand segments. The chopped strand segments may be compounded with a polymeric resin during an extrusion process and the resulting short fiber, compounded pellets. On the other hand, the continuous fiber strand packages may be used in continuous fiber thermoplastic composite fabrication using a long fiber thermoplastic (LFT) process to form continuous glass fiber reinforced polymer strands, rods, tapes, textiles or chopped long fiber reinforced pellets. These forms or structures, in turn, may be used to form hybrid composite articles.

In an example of the hybrid composite, a continuous glass fiber reinforced polymer matrix is used with magnesium or magnesium alloy comprising a bioresorbable polymer matrix and continuous bioresorbable reinforcing glass fiber, wherein the glass fiber has a tensile strength of about or over 2000 MPa. This enables obtaining a hybrid composite tensile strength of more than 450 MPa, and a composite flexural strength of more than 450 MPa. Thereby an orthopedic implant having composite tensile strength of more than 450 MPa, and a composite flexural strength of more than 450 MPa, is obtained.

The term "bioresorbable glass fiber reinforced polymer matrix" refers to any suitable depositable structure comprising the bioresorbable polymer matrix and the bioresorbable reinforcing fiber or fiber bundle in the structure. The bioresorbable reinforcing fiber may be discontinuous or continuous or mixture of those in the structure.

The weight ratio of the continuous bioresorbable reinforcing fiber or fiber bundle to the bioresorbable polymer is preferably such that the bioresorbable reinforcing fiber content is 1 to 99 weight-% of the total weight of the bioresorbable glass fiber reinforced polymer matrix, preferably 20 to 80 wt%, more preferably from 30 to 70 wt% and most preferably 40 to 60 wt%.

The smallest dimension of the bioresorbable glass fiber reinforced polymer matrix is preferably from 0.05 mm to 100 mm, more preferably 0.1 mm to 20 mm, even more preferably from 0.5 mm to 10.0 mm, most preferably from 0.8 mm to 5.0 mm.

The bioresorbable polymer may be a homopolymer or a copolymer, including random copolymer, block copolymer, or graft copolymer. Further, the bioresorbable polymer may be a linear polymer, a branched polymer, or a dendrimer. The bioresorbable polymers may be of natural or synthetic origin.

One or more of the following resorbable polymers, copolymers and terpolymers may be used as suitable bioresorbable polymers. For example, polylactides (PLA), poly-L-lactide (PLLA), poly-DL-lactide (PDLLA); polyglycolide (PGA); copolymers of glycolide, glycolide/trimethylene carbonate copolymers (PGA/TMC); other copolymers of PLA, such as lactide/tetramethylglycolide copolymers, lactide/trimethylene carbonate copolymers, lactide/d-valerolactone copolymers, lactide/e-caprolactone copolymers, L-lactide/DL-lactide copolymers, glycolide/L-lactide copolymers (PGA/PLLA), polylactide-co-glycolide; terpolymers of PLA, such as lactide/glycolide/trimethylene carbonate terpolymers, lactide/glycolide/ e - caprolactone terpolymers, PLA/polyethylene oxide copolymers; polydepsipeptides; unsymmetrically 3,6-substituted poly-1 ,4-dioxane-2,5-diones; polyhydroxyalkanoates; such as polyhydroxybutyrates (PHB); PHB/b-hydroxyvalerate copolymers (PHB/PHV); poly-b-hydroxypropionate (PHPA); poly-p-dioxanone (PDS); poly-d-valerolactone - poly-s-capralactone, Polytyrosines and its copolymers; polyacrylamides, poly(e-caprolactone-DL-lactide) copolymers; methylmethacrylate-N-vinylpyrrolidone copolymers; Polyvinylpyrrolidone and its copolymers; polyesteramides; polyacrylic acids, polybutylene succinate and its copolymers; polyoxazolines, Polyethylene glygols, polyesters of oxalic acid; polydihydropyrans; polyalkyl-2-cyanoacrylates; polyurethanes (PU); polyvinylalcohol (PVA); polypeptides; poly-b-malic acid (PMLA): poly-b-alkanoic acids; polycarbonates; polyorthoesters; polyphosphates; Polyphosphazenes; poly(ester anhydrides); biodegradable liquid crystal polymers; Xanthan Gum; Pectins; Dextran; Carrageenan; Guar Gum, Cellulose Ethers; Glucomannan; Sodium CMC; HPC; HPMC; and mixtures thereof; and natural polymers, such as sugars; Starch or Starch Based Derivatives, cellulose and cellulose derivatives, polysaccharides, collagen, chitin, chitosan, fibrin, hyalyronic acid, polypeptides and proteins. Mixtures and copolymers of any of the above-mentioned polymers and their various forms may also be used.

Particular examples of suitable bioresorbable polymers include, but are not limited to, polymers made from, obtained from or comprising, lactide, glycolide, caprolactone, valerolactone, carbonates, dioxanones, 6-valerolactone, ethylene glycol, ethylene oxide, esteramides, y-hydroxyvalerate, B-hydroxypropionate, alpha-hydroxyacid, hydroxybuterates, polyorthoesters, hydroxy alkanoates, tyrosine carbonates, polyimide carbonates, polyimino carbonates, polyurethanes, polyanhydrides, and copolymers and any combinations thereof. Suitable natural biodegradable polymers include collagen, chitin, chitosan, cellulose, polyaminoacids, polysaccharides, and copolymers, derivatives and combinations thereof.

The bioresorbable polymer is preferably selected from the group consisting of bioabsorbable polyesters, PLLA (poly-L-lactide), PDLLA (poly-DL-lactide), PLDLA, PGA (poly-glycolic acid), PLGA (poly-lactide-glycolic acid), PCL (polycaprolactone), PLLA-PCL and combinations thereof.

In addition to the bioresorbable polymer the bioresorbable glass fiber reinforced polymer matrix comprises a bioresorbable reinforcing glass fiber or fiber bundle.

The average fiber diameter of a single reinforcing fiber may be in the range of 1-100 micrometers, preferably 5-30 micrometers, more preferably 10-20 micrometers. This may be detected and determined microscopically.

In a preferred example, the bioresorbable reinforcing glass fiber or fiber bundle comprises or is comprised of phosphate or silica-based mineral compound. Most preferably the bioresorbable reinforcing fiber or fiber bundle is a melt derived silica-based bioresorbable glass fiber. In one example the bioresorbable glass is selected from silica, phosphate, boron and magnesium based bioresorbable glasses.

Typically, glass fibers are formed by attenuating streams of a molten glass material from a bushing. A sizing composition, or chemical treatment, may comprise lubricants, coupling agents, film-forming, binders, emulsifiers, surfactants, melt viscosity reducers, compatibilizers, adhesion promoters and anti-static agents, wetting agents, dispersing agents, catalysts, but not limited on those. Sizing's are typically applied to the fibers after they are drawn from the bushing. The sizing composition protects the fibers from inter-filament abrasion and promotes compatibility and adhesion between the glass fibers and the matrix in which the glass fibers are to be used. After the fibers are treated with the sizing composition, they may be dried and formed into a continuous fiber strand package or chopped into chopped strand segments. Glass fibers can then be used in the form of continuous or chopped filaments, strands, roving's, woven fabrics, nonwoven fabrics, meshes, and scrims in polymer matrix.

The bioresorbable glass fiber may comprise or have composition in the following wt% ranges (as a percent over the total weight of the glass fiber composition):

| | |
|---|---|
| SiO₂ | 40-90 wt%, |
| Na₂O | 1-30 wt%, |
| K₂O | 0-20 wt %, |
| CaO | 5-30 wt%, |
| MgO | 0-20 wt%, |
| P₂O₅ | 0-20 wt%, |
| B₂O₃ | 0-20 wt%, |
| Al₂O₃ | 0-10 wt%, |
| CaF₃ | 0-25 wt%, |
| SrO | 0-10 wt%, and |
| Li₂O | 0-5 wt%. |

In a first example the bioresorbable glass fiber has composition in the following wt% ranges:

| | |
|---|---|
| SiO₂ | 50-75 wt%, |
| Na₂O | 5-20 wt%, |
| K₂O | 0-10 wt%, |
| CaO | 5-25 wt%, |
| MgO | 0-10 wt%, |
| P₂O₅ | 0.5-5 wt%, |
| B₂O₃ | 0-15 wt%, |
| Al₂O₃ | 0-5 wt%, and |
| SrO | 0-5 wt%. |

In a second example the melt derived bioabsorbable glass fiber has composition in the following wt% ranges:

| | |
|---|---|
| SiO₂ | 60-72 wt%, |
| Na₂O | 10-20 wt%, |
| K₂O | 0.1-10 wt%, |
| CaO | 5-15 wt%, |
| MgO | 1-10 wt%, |
| P₂O₅ | 0.5-2 wt%, |
| SrO | 0-3 wt%, and |
| B₂O₃ | 0-10 wt%. |

The sum of the ingredients of the compositions sums up to 100%.

Therefore, the present application also discloses a bioresorbable glass fiber and a composition for forming the bioresorbable glass fiber. As used herein, the term "bioresorbable glass fiber" is meant to denote that the glass fiber can be dissolved and/or degraded by the action of water or other natural agents. The bioresorbable fibers may be used as reinforcement for composite parts. The bioresorbable reinforcing glass fiber may be bioactive and/or osteoconductive depending on the glass composition

The bioresorbable glass fiber may be used in conjunction with bioresorbable polymers and the magnesium alloy to form a hybrid composite product that is naturally non-toxic, biocompatible, bioresorbable, biosoluble and biodegradable over a period of time. The bioresorbable fibers have mechanical properties comparative to conventional, non-soluble glass fibers, have a slow to high rate of dissolution in an aqueous medium i.e. low hydrolytic strength and are easily fiberized.

In an advantageous example of the hybrid composite, the bioresorbable glass fiber reinforced polymer matrix comprises a bioresorbable polymer which is preferably selected from the group consisting of bioresorbable polyesters, PLLA (poly-L-lactide), PDLLA (poly-DL-lactide), PLDLA, PGA (poly-glycolic acid), PLGA (poly-lactide-glycolic acid), PCL (polycaprolactone), PLLA-PCL and combinations thereof; and the bioresorbable reinforcing glass fiber or fiber bundle comprised of a melt derived bioresorbable glass fiber. Preferably the composition of the melt derived bioresorbable glass fiber is as defined above.

In addition to polymer matrix and the bioresorbable reinforcing glass fiber or fiber bundle the bioresorbable glass fiber reinforced polymer matrix may also comprise a bioresorbable sizing in bioresorbable reinforcing glass fiber for improving adhesion between inorganic glass and organic polymer phase, improve processability of the bioresorbable glass fiber reinforced polymer matrix and fiber dispersion in polymer matrix.

Additionally or alternatively the bioresorbable glass fiber reinforced polymer matrix may also comprise one or more reinforcements or filler materials besides of bioresorbable glass fiber, such as ceramic particles (e.g. tricalcium phosphate particles), antimicrobial agents, bioactive agents, active pharmaceutical ingredients, other reinforcing fibers may be comprise other bioresorbable glass composition or glass-like materials, a ceramic, a mineral composition such as hydroxyapatite, tricalcium phosphate, calcium sulfate or calcium phosphate, a cellulosic material, or any other continuous fiber known in the art to increase the mechanical properties of a bioresorbable polymer. The continuous reinforcing fiber may also be a bioresorbable polymer itself.

One effect of the implants or a structural part is their strength and feasible manufacturing. An implant or structural part thereof can be manufactured by arranging any reinforcing form of magnesium or magnesium alloy and bioresorbable glass fibers in a bioresorbable polymer matrix and using one or more of suitable of polymer or composite processing device(s) or equipment, such as a mechanical processing device, for example an open or closed batch mixer or kneader, extruder including coextrusion and thermoplastic pultrusion, injection molding machine including insert molding, reactive injection molding (RIM), lamination, calenders, transfer molding, compression molding, mechanical machining, pultrusion, solvent casting, 3D printing, filament winding, automated tape lay-up, automated fiber placement or other standard melt processing or melt mixing equipment known in the field and including combinations of aforementioned, producing and/or shaping into an implant or structural part having a desired orientation and ratio of the magnesium or magnesium alloys and continuous bioresorbable glass fibers and/or chopped/cut fibers and/or woven, non-woven mats/textiles.

One effect of the implants according to the present application, both the composite implants and the non-composite implants, is that they disappear from the body by degradation without giving rise to adverse events such as too fast hydrogen evolution.

Depending on the application and purpose of the implant material, the implants, in addition to being biocompatible, also exhibit controlled resorption in the mammalian body. The optimal resorption rate is directly proportional to the renewal rate of the tissue in the desired implantation location. In the case of bone tissue, a considerable proportion of the implant is preferably resorbed/decomposed within 12 to 24 months in the tissue. In cases where more physical support to the healing tissues is desirable, the resorption rate might be several months or even several years. Furthermore, mechanically stable implants having specific structures, such as relatively thin walls, tubes or other parts, for example cannulas, catheters and stents, can be provided.

The implants may be used in medical treatment methods, such as methods comprising inserting the implant into tissue, such as into a subject, such as a patient. For example, such a method may comprise
- preferably recognizing a subject in need of treatment or therapy,
- providing the implant,
- inserting the implant into the subject.

The subject may be human or animal subject. The need of therapy may be caused by a damage in a bone or other applicable tissue. For example the subject may suffer from a bone fracture or other damage, or other applicable condition, such as ones disclosed herein.

### Examples

### Example 1: In vitro comparison between pure MgCaZn alloy and MgCaZn alloy with 69 ppm Fe and 228 ppm Zr

The *in vitro* degradation of the MgCaZn-alloy and MgCaZnFeZr-alloy were evaluated. The sample masses were measured and reported weekly till 12 weeks. The composition of the studied materials were (by weight): a) MgZnCa-alloy: 99% Mg, 0.5% Zn and 0.5% Ca, and b) MgZnCaFeZr-alloy: 99 %Mg, 0.5 % Zn, 0.5 % Ca, 69 ppm Fe and 228 ppm Zr. The test articles were discs with the following dimensions: diameter 6 mm and thickness 1 mm. They were machined from the 8 mm raw material bars. The discs also had a small hole so that they can be hung in the sample containers. The number of parallel test articles was three for all the tests. In each time point samples were measured and put back to the hydrolysis after testing. The test articles were handled carefully with protective gloves in order to protect the coating and to decrease the risk of contamination.

The study was partly performed according to standards ASTM F1635-16, ISO 13781:2017 and ASTM F3268-18a. The detailed *in vitro* test conditions and apparatus are described in Table 1 below.

**Table 1.**

| | |
|---|---|
| Method | ASTM F1635-16, ISO 13781:2017 and ASTM F3268-18a |
| Laboratory | R&D Lab |
| Soaking solution | Sorensen buffer solution. |
| | Soaking solution is changed every 4 weeks and whenever needed to keep the pH in tolerances. |
| Sample container | 500 ml plastic container |
| Soaking solution-to-sample mass ratio | At least 30: 1 (ml:g) |
| | Test article mass: about 50 mg |
| | Solution volume: about 500 ml |
| Conditions | Temperature: 37±1°C |
| | pH: 7.4 ± 0.2 |
| Constant temperature oven | D-99 Memmert UFE800, D-99 or D-18 |
| pH meter | D-25 WTW pH-meter |

The test articles were stringed with a suture in order to fully immerse them in the buffer solution without the test articles touching the bottom of the container. The pH of the buffer solution was monitored and possible clouding in it was checked weekly. The solution was changed every 4 weeks and whenever needed to keep the pH in tolerances.

Mass loss monitoring included the following steps
- Weigh the initial mass before hydrolysis and record the results.
- At each test point dry the samples with non-pilling cloth and weigh the samples
- At the final time point dry the specimens in vacuum oven.
- Record the wet and dry weight

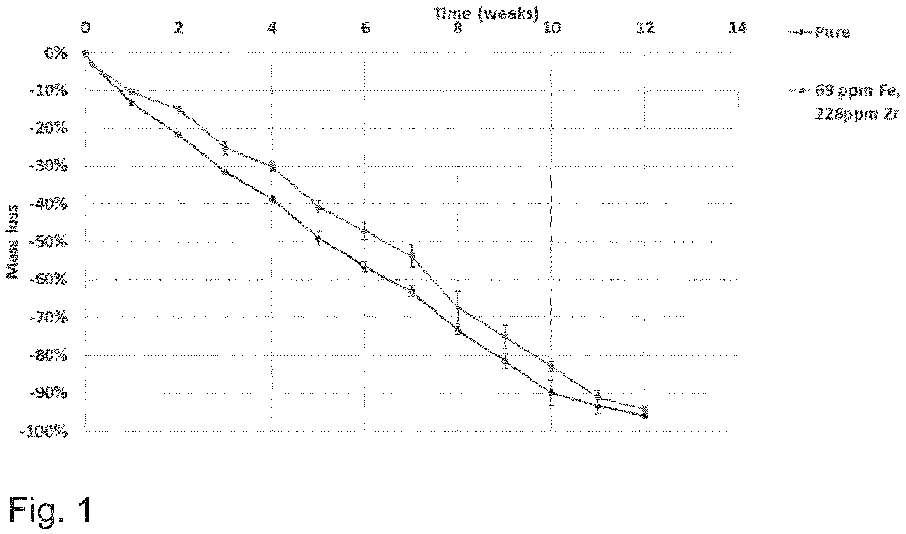

Result are presented in Figure 1, which shows that corrosion rates of the MgZnCa-alloy with 0.5% Zn and 0.5% Ca, and MgZnCaFeZr-alloy with 0.5% Zn, 0.5% Ca, 69 ppm Fe and 228 ppm Zr are comparable or that the MgCaZnFeZr-alloy with 69 ppm Fe and 228 ppm Zr is corroding even slightly slower rate.

### Example 2: Heat treatment (dry heat sterilization) effect on mechanical properties

Heat treatment experiment was carried out with 6 mm and 7 mm MgCaZnFeZr-alloy (with 69 ppm Fe and 228 ppm Zr) bars using Stericell dry heat sterilizer. Used parameters were temperature (160°C, 180°C and 200°C) and time (3h). In each parameter combinations six parallel samples were used and after treatment bending and shear tests were performed according standards ASTM D790-17 and ASTM B769-11(16) accordingly. The flexural strength and modulus were outcome from the bending test and the shear strength from the shear test.

Results are presented in Figure 2, which shows that heat treatment clearly improved mechanical properties of the Mg-alloy at each temperature, but the best results were achieved at 160°C.

### Example 3: Grain size effect on mechanical properties

Effect of the grain size was studied with two different material six samples each. Both samples had the same alloy composition (99% Mg, 0.5% Ca, 0.5% Zn, 69 ppm Fe, 228 ppm Zr) and they were both treated in the same temperature (160°C) and during the same time (2 hours).

For the grain size analyses samples were prepared by using following method:
- Cleaning and polishing an oxide polishing suspension (+ammonia)
- Etching by subsequently immersed (5 - 10 s) in the etching solution (40 ml solution 1 (100 ml ethanol + 5.6 g of picric acid) + 10 ml solution 2 (5 ml of acetic acid + 5 ml deionized water)
- Finally the samples were cleaned in isopropanol and dried
- Grain sizes were analysed using stereomicroscope and the analyses were according the ASTM E112 standard.

Smaller grain size (≤20µm) alloy had higher flexural strength, and modulus as well as shear strength as presented in the Figure 3.

### Example 4: Manufacturing of compression screw

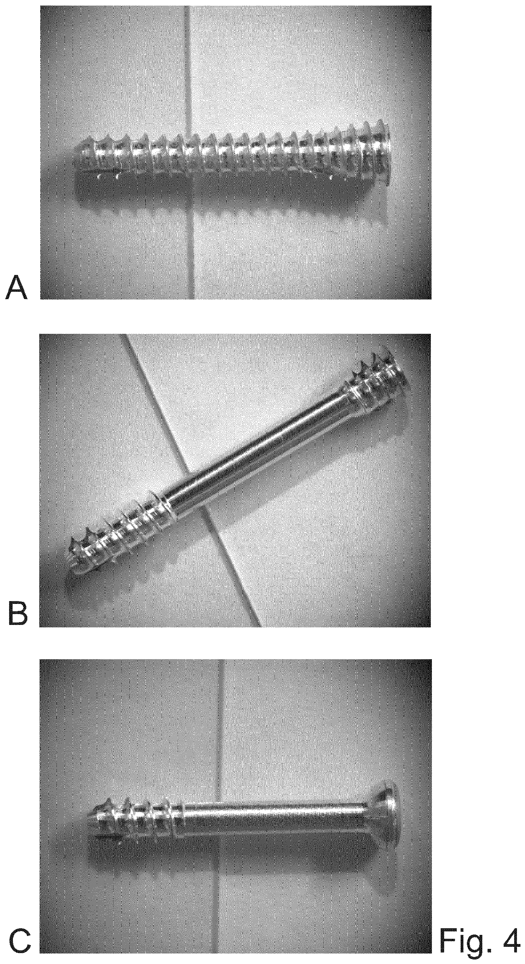

Three different cannulated compression screw designs, as shown in figures 4A-C, were manufactured and tested. Figure 4A shows a conical headless screw, with three different thread sections: distal, where the pitch is the biggest, middle, and a proximal section with the smallest pitch (FFT). Figure 4B shows a headless screw with threaded distal and proximal ends; the distal end having a bit bigger pitch and smaller diameter than the proximal end (FC). Figure 4C shows a Lag-screw with the head and threadless, lag section, and threaded distal end (FL).

Screws were machined from the extruded and cold drawn bar, having a diameter of 7 mm. Machining was carried out with a 5-axis swiss-machine. All screw designs were manufactured in diameters from 2 to 4 mm and lengths from 10 to 55 mm. After machining, the screws were cleaned in an ultrasonic cleaner with ethanol, packed into plastic pouches, and dry heat sterilized (2 hours in 160°C). After the sterilization, the screws were mechanically and biomechanically tested.

The torsional test results of the Ø3.5 x 40 mm FC-screw (figure 4B) are presented in Table 2 below.

**Table 2**

| **Sample** | **Torque Yield (Ncm)** | **Max load (Ncm)** | **Rotation in Max load (deg.)** |
|---|---|---|---|
| FC-1 | 29 | 46 | 215 |
| FC-2 | 27.5 | 43.4 | 210 |
| FC-3 | 28 | 36 | 110 |
| AVG | **28.2** | **41.8** | **178.3** |
| STDV | 0.8 | 5.2 | 59.2 |

The biomechanical pullout test results of the Ø2.0 x 8 mm FL-screw (figure 4C) are presented below. Screws were inserted into the porcine bone and then pulled out, and the maximum load was recorded. The results are presented in Table 3.

**Table 3.**

| **Sample** | **Max load (N)** |
|---|---|
| FL-1 | 202 |
| FL-2 | 130 |
| AVG | **166** |

These results are comparable with titanium screws and clearly higher than with absorbable polymer screws.

### Example 5: Plastic deformation (cold drawing) effect on machinability and efficiency (lower scrap percentage)

To obtain acceptable diameter tolerances, straightness, roundness, and surface quality from extruded bars a cold drawing process was used. The Mg-alloy bar was drawn through the die, which was slightly smaller than the Mg-alloy bar. This process was repeated several times until diameter, straightness, roundness, and surface quality were on the preferable level. In each process step, the die was slightly smaller than the previous.

When changed from straight extruded bars to cold drawn bars, the scrap percentage dropped from over 50% to less than 5%, and the same time average machining time for a single screw dropped over 30%.

### Example 6. Cytotoxicity of the MqCaZnZrFe-alloy - Standard (ISO 10993) acute cytotoxicity test for medical devices

BJ Neutral Red uptake (NRU) assay was used to predict acute toxicity of medical device extracts. Cytotoxicity of medical device is a part of biological evaluation process of medical devices. The test complies with the ISO standard 10993 "Biological evaluation of medical devices, Part 5: Tests for in vitro cytotoxicity (ISO 10993-5) and Part 12: Sample preparation and reference materials (ISO 10993-12).

### Assay Conditions

- NRU is an *in vitro* basal cytotoxicity assay that is based on the ability of viable cells to incorporate and bind neutral red, a supravital dye after the cells are exposed to test material extracts.
- The test begins with extraction of test material. Extraction is performed at 37 °C for 72 h in cell growth medium containing 10% serum in order to release both polar and non-polar substances to the medium.
- Cytotoxicity testing is performed using undiluted (100%) test material extract and 1:2 dilution of the extract with 6 parallels of each to determine whether the extract reduces NRU compared to negative (untreated) control. Alternatively, the test can be performed in a dose-response format using 8 different concentrations of test material extracts with 6 parallels of each to determine the IC50 concentration (dilution %)
- Exposure time 48 hrs at 37°C, 5.0% CO₂
- Positive and negative controls are run in each test

The results are calculated as % cell viability as compared to untreated control (BJ cell culture medium) and classified as cytotoxic or non-cytotoxic.

The % viabilities of BJ cells exposed to 100 % test item extracts as compared to the controls (VC) are presented in Table 4.

**Table 4. The effect of 100% test item extracts (72 hrs and 24 hrs extraction at +37°C) on BJ cell viability, and the classification of test items to cytotoxic or non-cytotoxic.**

| | **Test item** | **% Cell viability vs negative control after exposure to 100% extracts** | | **Classification *** |
|---|---|---|---|---|
| | | **Mean** | **5D** | |
| 1 | 3-day extraction | 87.23 | 7.93 | non-cytotoxic |
| 2 | 1-day extraction | 87.98 | 1.83 | non-cytotoxic |

| | | | | |
|---|---|---|---|---|
| *The test item extract is considered cytotoxic, If the viability of BJ cells after the exposure to 100% extracts is less than 70% as compared to negative (vehicle) controls, and non-cytotoxic if the viability is ≥70%. | | | | |

The dose response data is shown in Table 5. IC₅₀ value cannot be calculated, since no cytotoxic effect was seen.

**Table 5. Dilution series of test item extracts (72 hrs and 24 hrs extraction at +37°C). The effect on BJ cell viability is calculated towards the negative control.**

| extract% | 3-day extraction | | | 1-day extraction | | |
|---|---|---|---|---|---|---|
| | Mean | stdev | N | Mean | stdev | N |
| 0.00 | 100.00 | 4.29 | 12 | 100.00 | 5.17 | 12 |
| 0.50 | 96.66 | 2.88 | 6 | 103.27 | 4.75 | 6 |
| 1.00 | 95.60 | 3.60 | 6 | 102.24 | 5.06 | 6 |
| 2.20 | 101.40 | 6.17 | 6 | 102.65 | 5.39 | 6 |
| 4.70 | 124.07 | 11.11 | 6 | 109.04 | 5.66 | 6 |
| 10.10 | 137.11 | 4.96 | 6 | 137.40 | 14.83 | 6 |
| 21.60 | 113.68 | 9.73 | 6 | 132.86 | 7.38 | 6 |
| 46.50 | 109.93 | 22.71 | 6 | 116.92 | 13.51 | 6 |
| 100.00 | 87.23 | 7.93 | 6 | 87.98 | 1.83 | 5 |

As a conclusion MgCaZnFeZr-alloy is not cytotoxic and even increased cell viability with dilutions.

## Claims

1. An implant comprising biodegradable magnesium alloy consisting of magnesium and
- Ca in the range of 0.3-2 wt%,
- Zn in the range of 0.5-6 wt%,
- Fe in the range of 50-100 ppm, and
- Zr in the range of 100-900 ppm and total impurities including Fe and Zr in the range of 400-1000 ppm, optionally wherein total impurities comprise Mn, Cu, Ni, Al, Pb, Cd, and rare earth elements below 800 ppm, wherein the magnesium alloy has an average grain size of 40 µm or less.

2. The implant of claim 1, comprising biodegradable magnesium alloy consisting of magnesium and
- Ca in the range of 0.4-0.7 wt%,
- Zn in the range of 0.5-0.7 wt%,
- Fe in the range of 50-100 ppm, and
- Zr in the range of 100-900 ppm and total impurities including Fe and Zr in the range of 400-1000 ppm.

3. The implant of claim 1 or 2, wherein the magnesium alloy has an average grain size of 20 µm or less.

4. The implant of any of the preceding claims, wherein the ratio of weight percentages Ca:(Zn-0.2) is more than 0.1.

5. The implant of any of the preceding claims, wherein the ratio of weight percentages of Fe to Zr is in the range of 1:1 to 1:20.

6. The implant of any of the preceding claims, wherein the magnesium alloy comprises total impurity of Mn, Cu, Ni, Al, Pb, Cd, and rare earth elements below 600 ppm.

7. The implant of any of the preceding claims, wherein the magnesium alloy and/or the implant does not contain rare earth metals, such as yttrium (Y), gadolinium (Gd) and/or neodymium (Nd).

8. The implant of any of the preceding claims, wherein it is in a form of a composite with one or more biodegradable polymer(s), glass fiber(s), bioglass and/or ceramic material(s) such as hydroxyapatite, tricalcium phosphate, calcium carbonate, calcium sulphate, and/or is doped with silicone or magnesium.

9. The implant of any of the preceding claims, wherein it is an orthopedic implant.

10. The implant of any of the preceding claims, comprising a screw, a plate, a pin, a tack or a nail, such as intramedullary nail, preferably for the fixation of bone fractures and/or osteotomies to immobilize the bone fragments for healing; a suture anchor, a tack, a bolt, a clamp, a clip, a staple, a mesh, a scaffold, a cage, a Kirschner wire, a stent or other device preferably for bone-to-bone, soft tissue-to-bone, soft tissue-into-bone and/or soft tissue-to-soft tissue fixation; such as a device used for supporting tissue or bone healing or regeneration; or cervical wedges and lumbar cage or plate, or a screw for vertebral fusion and other operations in spinal surgery.

11. An orthopedic implant comprising the implant of any of the preceding claims, which orthopedic implant is an elongated bone fixation device having a length:diameter ratio of 2 or more and which is preferably selected from a screw, an intramedullary nail, a pin and a Kirschner wire, and wherein the magnesium alloy has an average grain size of 40 µm or less.

12. A method for preparing the implant of any of the preceding claims, the method comprising
- providing a biodegradable magnesium alloy object, preferably an elongated object, consisting of magnesium and
- Ca in the range of 0.3-2 wt%,
- Zn in the range of 0.5-6 wt%,
- Fe in the range of 50-100 ppm, and
- Zr in the range of 100-900 ppm and total impurities including Fe and Zr in the range of 400-1000 ppm, optionally wherein total impurities comprise Mn, Cu, Ni, Al, Pb, Cd, and rare earth elements below 800 ppm, and
having an average grain size of 40 µm or less, and
- forming the object into the implant.

13. The method of claim 12, comprising
- providing biodegradable magnesium alloy,
- heating the magnesium alloy to above the recrystallization temperature of the magnesium alloy, such as to a temperature of at least 200°C, for example to 250-400°C,
- forming the heated magnesium alloy into the object having the average grain size of 40 µm or less, preferably in a plastic deformation and/or grain refinement process.

14. The method of claim 12 or 13, comprising heating the biodegradable magnesium alloy at 160-200°C, such as 160-180°C, for at least 30 minutes, such as 30-180 minutes, before and/or after forming the biodegradable magnesium alloy into the implant.

15. The method of any of the claims 12-14, wherein the forming the object into an implant comprises processing the object with one or more of the following:
- mechanical machining,
- laser machining,
- wroughting,
- water jet processing,
- additive manufacturing, such as comprising providing the biodegradable magnesium alloy as powder, granules or as a wire, and
- injection molding, such as by thixotropic molding, liquid metal molding or metal injection molding, preferably by using a binder,
to form the implant.

## Patentansprüche

1. Implantat mit einer biologisch abbaubaren Magnesiumlegierung, bestehend aus
Magnesium und
- Ca im Bereich von 0,3-2 Gew.-%,
- Zn im Bereich von 0,5-6 Gew.-%,
- Fe im Bereich von 50-100 ppm, und
- Zr im Bereich von 100-900 ppm und Gesamtverunreinigungen einschließlich Fe und Zr im Bereich von 400-1000 ppm, wobei wahlweise die Gesamtverunreinigungen Mn, Cu, Ni, Al, Pb, Cd und Seltenerdelemente unter 800 ppm umfassen, wobei die Magnesiumlegierung eine durchschnittliche Korngröße von 40 µm oder weniger aufweist.

2. Implantat nach Anspruch 1, umfassend eine biologisch abbaubare Magnesiumlegierung, bestehend aus Magnesium und
- Ca im Bereich von 0,4-0,7 Gew.-%,
- Zn im Bereich von 0,5-0,7 Gew.-%,
- Fe im Bereich von 50-100 ppm, und
- Zar im Bereich von 100-900 ppm und Gesamtverunreinigungen einschließlich Fe und Zr im Bereich von 400-1000 ppm.

3. Das Implantat nach Anspruch 1 oder 2, wobei die Magnesiumlegierung eine durchschnittliche Korngröße von 20 µm oder weniger aufweist.

4. Das Implantat nach einem der vorhergehenden Ansprüche, wobei das Verhältnis der Gewichtsprozente Ca: (Zn-0,2) mehr als 0,1 beträgt.

5. Das Implantat nach einem der vorhergehenden Ansprüche, wobei das Verhältnis der Gewichtsprozente von Fe zu Zr im Bereich von 1:1 bis 1:20 liegt.

6. Das Implantat nach einem der vorhergehenden Ansprüche, wobei die Magnesiumlegierung eine Gesamtverunreinigung von Mn, Cu, Ni, Al, Pb, Cd und Seltenerdelementen unter 600 ppm aufweist.

7. Das Implantat nach einem der vorhergehenden Ansprüche, wobei die Magnesiumlegierung und/oder das Implantat keine Seltenerdmetalle, wie Yttrium (Y), Gadolinium (Gd) und/oder Neodym (Nd), enthält.

8. Das Implantat nach einem der vorhergehenden Ansprüche, wobei es in Form eines Verbundstoffs mit einem oder mehreren biologisch abbaubaren Polymeren, Glasfasern, Bioglas und/oder keramischen Materialien, wie Hydroxylapatit, Tricalciumphosphat, Calciumcarbonat, Calciumsulfat, vorliegt und/oder mit Silikon oder Magnesium dotiert ist.

9. Das Implantat nach einem der vorhergehenden Ansprüche, wobei es sich um ein orthopädisches Implantat handelt.

10. Implantat nach einem der vorhergehenden Ansprüche, umfassend eine Schraube, eine Platte, einen Stift, eine Zwecke oder einen Nagel, wie z.B. einen Marknagel, vorzugsweise zur Fixierung von Knochenbrüchen und/oder Osteotomien, um die Knochenfragmente für die Heilung zu immobilisieren; einen Nahtanker, eine Zwecke, einen Bolzen, eine Klammer, einen Clip, eine Klammer, ein Netz, ein Gerüst, einen Käfig, einen Kirschnerdraht, einen Stent oder eine andere Vorrichtung, vorzugsweise für die Fixierung von Knochen an Knochen, von Weichgewebe an Knochen, von Weichgewebe in Knochen und/oder von Weichgewebe an Weichgewebe; wie eine Vorrichtung, die zur Unterstützung der Heilung oder Regeneration von Gewebe oder Knochen verwendet wird, oder zervikale Keile und lumbale Käfige oder Platten oder eine Schraube für die Wirbelfusion und andere Operationen in der Wirbelsäulenchirurgie.

11. Orthopädisches Implantat, umfassend das Implantat nach einem der vorhergehenden Ansprüche, wobei das orthopädische Implantat eine längliche Knochenfixierungsvorrichtung mit einem Verhältnis von Länge zu Durchmesser von 2 oder mehr ist und vorzugsweise aus einer Schraube, einem Marknagel, einem Stift und einem Kirschnerdraht ausgewählt ist, und wobei die Magnesiumlegierung eine durchschnittliche Korngröße von 40 um oder weniger aufweist.

12. Verfahren zur Herstellung des Implantats nach einem der vorhergehenden Ansprüche, wobei das Verfahren Folgendes umfasst
- Bereitstellen eines biologisch abbaubaren Gegenstands aus einer Magnesiumlegierung, vorzugsweise eines länglichen Gegenstands, bestehend aus Magnesium und
- Ca im Bereich von 0,3-2 Gew.-%,
- Zn im Bereich von 0,5-6 Gew.-%,
- Fe im Bereich von 50-100 ppm, und
- Zr im Bereich von 100-900 ppm und Gesamtverunreinigungen einschließlich Fe und Zr im Bereich von 400-1000 ppm, wobei gegebenenfalls die Gesamtverunreinigungen
Mn, Cu, Ni, Al, Pb, Cd und Seltenerdelemente unter 800 ppm umfassen, und
eine durchschnittliche Korngröße von 40 um oder weniger aufweisen, und
- Formen des Objekts zu dem Implantat.

13. Verfahren nach Anspruch 12, welches Folgendes umfasst
- Bereitstellen einer biologisch abbaubaren Magnesiumlegierung,
- Erhitzen der Magnesiumlegierung auf eine Temperatur oberhalb der Rekristallisationstemperatur der Magnesiumlegierung, wie auf eine Temperatur von mindestens 200°C, z.B. auf 250-400°C,
- Formen der erhitzten Magnesiumlegierung zu dem Gegenstand mit einer durchschnittlichen Korngröße von 40 um oder weniger, vorzugsweise in einem plastischen Verformungs- und/oder Kornverfeinerungsverfahren.

14. Das Verfahren nach Anspruch 12 oder 13, umfassend das Erhitzen der biologisch abbaubaren Magnesiumlegierung auf 160-200°C, wie 160-180°C, für mindestens 30 Minuten, wie 30-180 Minuten, vor und/oder nach dem Formen der biologisch abbaubaren Magnesiumlegierung zu dem Implantat.

15. Das Verfahren nach einem der Ansprüche 12 bis 14, wobei das Formen des Objekts zu einem Implantat die Verarbeitung des Objekts mit einem oder mehreren der folgenden Verfahren umfasst:
- mechanische Bearbeitung,
- Laserbearbeitung,
- Kneten,
- Wasserstrahlbearbeitung,
- additive Fertigung, beispielsweise durch Bereitstellen der biologisch abbaubaren Magnesiumlegierung als Pulver, Granulat oder als Draht, und
- Spritzgießen, beispielsweise durch thixotropes Gießen, Flüssigmetallgießen oder Metallspritzgießen, vorzugsweise unter Verwendung eines Bindemittels,
um das Implantat zu formen.

## Revendications

1. Implant comprenant un alliage de magnésium biodégradable constitué de
magnésium et de
- Ca dans la plage de 0,3 à 2 % en poids,
- Zn dans la plage de 0,5 à 6 % en poids,
- Fe dans la plage de 50 à 100 ppm, et
- Zr dans la plage de 100 à 900 ppm et des impuretés totales, y compris Fe et Zr, dans la plage de 400 à 1 000 ppm, éventuellement dans lequel les impuretés totales comprennent Mn, Cu, Ni, Al, Pb, Cd et des éléments de terres rares inférieures à 800 ppm, dans lequel l'alliage de magnésium a une taille de grain moyenne inférieure ou égale à 40 µm.

2. Implant selon la revendication 1, comprenant un alliage de magnésium biodégradable constitué de magnésium et de
- Ca dans la plage de 0,4 à 0,7 % en poids,
- Zn dans la plage de 0,5 à 0,7 % en poids,
- Fe dans la plage de 50 à 100 ppm, et
- Zr dans la plage de 100 à 900 ppm et des impuretés totales, y compris Fe et Zr, dans la plage de 400 à 1 000 ppm.

3. Implant selon la revendication 1 ou 2, dans lequel l'alliage de magnésium a une taille de grain moyenne inférieure ou égale à 20 µm.

4. Implant selon l'une quelconque des revendications précédentes, dans lequel le rapport des pourcentages en poids Ca:(Zn-0,2) est supérieur à 0,1.

5. Implant selon l'une quelconque des revendications précédentes, dans lequel le rapport des pourcentages en poids entre Fe et Zr est compris dans la plage de 1:1 à 1:20.

6. Implant selon l'une quelconque des revendications précédentes, dans lequel l'alliage de magnésium comprend une impureté totale de Mn, Cu, Ni, Al, Pb, Cd et des éléments de terres rares inférieure à 600 ppm.

7. Implant selon l'une quelconque des revendications précédentes, dans lequel l'alliage de magnésium et/ou l'implant ne contiennent pas de métaux de terres rares, tels que l'yttrium (Y), le gadolinium (Gd) et/ou le néodyme (Nd).

8. Implant selon l'une quelconque des revendications précédentes, dans lequel l'implant se présente sous la forme d'un composite doté d'un ou plusieurs polymères biodégradables, fibres de verre, bioverres et/ou matériaux céramiques tels que l'hydroxyapatite, le phosphate tricalcique, le carbonate de calcium, le sulfate de calcium et/ou est dopé avec du silicone ou du magnésium.

9. Implant selon l'une quelconque des revendications précédentes, dans lequel l'implant est un implant orthopédique.

10. Implant selon l'une quelconque des revendications précédentes, comprenant une vis, une plaque, une broche, une punaise ou un clou, tel qu'un clou intramédullaire, de préférence pour la fixation de fractures osseuses et/ou d'ostéotomies pour immobiliser les fragments osseux pour la cicatrisation ; une ancre de suture, une punaise, un boulon, une pince, un clip, une agrafe, un treillis, un échafaudage, une cage, un fil de Kirschner, un stent ou un autre dispositif, de préférence pour la fixation os à os, tissu mou à os, tissu mou dans l'os et/ou tissu mou à tissu mou ; tel qu'un dispositif utilisé pour stimuler la cicatrisation ou la régénération des tissus ou des os ; ou des cales cervicales et une cage ou une plaque lombaire, ou une vis pour la fusion vertébrale et d'autres opérations en chirurgie du rachis.

11. Implant orthopédique comprenant l'implant selon l'une quelconque des revendications précédentes, lequel implant orthopédique est un dispositif de fixation osseuse allongé ayant un rapport longueur:diamètre de 2 ou plus et qui est de préférence choisi parmi une vis, un clou intramédullaire, une broche et un fil de Kirschner, et dans lequel l'alliage de magnésium a une taille de grain moyenne inférieure ou égale à 40 µm.

12. Procédé pour préparer l'implant selon l'une quelconque des revendications précédentes, le procédé comprenant
- la fourniture d'un objet biodégradable en alliage de magnésium, de préférence un objet allongé, constitué de magnésium et de
- Ca dans la plage de 0,3 à 2 % en poids,
- Zn dans la plage de 0,5 à 6 % en poids,
- Fe dans la plage de 50 à 100 ppm, et
- Zr dans la plage de 100 à 900 ppm et des impuretés totales, y compris Fe et Zr, dans la plage de 400 à 1 000 ppm, éventuellement dans lequel les impuretés totales comprennent Mn, Cu, Ni, Al, Pb, Cd et des éléments de terres rares inférieurs à 800 ppm, et
ayant une taille de grain moyenne inférieure ou égale à 40 µm, et
- la transformation de l'objet en l'implant.

13. Procédé selon la revendication 12, comprenant
- la fourniture d'un alliage de magnésium biodégradable,
- le chauffage de l'alliage de magnésium au-dessus de la température de recristallisation de l'alliage de magnésium, par exemple jusqu'à une température d'au moins 200°C, par exemple entre 250 et 400°C,
- la transformation de l'alliage de magnésium chauffé en un objet ayant une taille de grain moyenne inférieure ou égale à 40 µm, de préférence lors d'un processus de déformation plastique et/ou de raffinement des grains.

14. Procédé selon la revendication 12 ou 13, comprenant le chauffage de l'alliage de magnésium biodégradable à 160-200°C, tel que 160-180°C, pendant au moins 30 minutes, tel que 30 à 180 minutes, avant et/ou après la transformation de l'alliage de magnésium biodégradable en l'implant.

15. Procédé selon l'une quelconque des revendications 12 à 14, dans lequel la transformation de l'objet en implant comprend le traitement de l'objet par une ou plusieurs des techniques suivantes :
- usinage mécanique,
- usinage laser,
- forgeage,
- traitement au jet d'eau,
- fabrication additive, telle que comprenant la fourniture de l'alliage de magnésium biodégradable sous forme de poudre, de granulés ou de fil, et
- moulage par injection, tel que par moulage thixotropique, moulage de métal liquide ou moulage par injection de métal, de préférence à l'aide d'un liant, pour former l'implant.
